# EUROPEAN PATENT APPLICATION

(11) **EP 4 781 989 A1**
(43) Date of publication of application: **29.07.2026**
(21) Application number: 23856747.3
(22) Date of filing: 18.09.2023
(51) Int. Cl.: A61K 31/496, A61K 9/14, A61K 31/5415, A61K 47/04, A61P 31/10

(54) **POSACONAZOLE SOLID DISPERSION AND PREPARATION METHOD THEREFOR**

(71) Applicant: BEIJING DELIVERY PHARMACEUTICAL TECHNOLOGY CO., LTD, Yanqing District, Beijing 102101 (CN)
(72) Inventor: WU, Cuishuan, Beijing 102101 (CN); WANG, Xiaoxu, Beijing 102101 (CN); WANG, Shengnan, Beijing 102101 (CN); XIAO, Jiahao, Beijing 102101 (CN)
(74) Representative: Nederlandsch Octrooibureau
(86) International application number: PCT/CN2023/119464
(87) International publication number: WO 2024/041662

(57) **Abstract**

A posaconazole solid dispersion having improved dissolution properties and bioavailability, and a preparation method therefor. The solid dispersion contains posaconazole, silicon dioxide, sodium dodecyl sulfate (SDS) and hydroxypropyl methylcellulose acetate succinate (HPMCAS), wherein the weight ratio of SDS to posaconazole is ≥ 1:10, the weight ratio of posaconazole to silicon dioxide is 1:0.8 to 1:1, the weight ratio of posaconazole to HPMCAS is 1:2 to 1:5; the silicon dioxide is selected from SYSLSIA350FCP, SYLORD244FP, SYLORD3050 and a mixture thereof at any ratio, and the HPMCAS is selected from HPMCAS-MG, HPMCAS-LF, HPMCAS-MF, HPMCAS-LG and a mixture thereof at any ratio.

## Description

### Technical Field

The present invention belongs to the field of pharmaceutical formulations. The present disclosure specifically relates to a posaconazole solid dispersion with improved dissolution and bioavailability and a preparation method therefor.

### Background

Posaconazole is a second-generation triazole antifungal agent having the following formula:

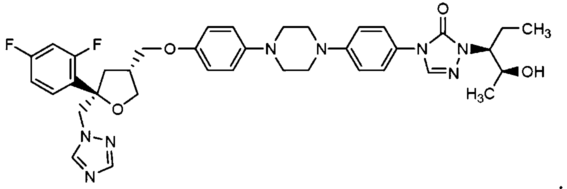

With the increasing number of high-risk patients such as those undergoing cancer chemotherapy, transplantation, HIV/AIDS infection, and diabetes, the incidence of invasive fungal infections continues to rise, with a mortality rate of up to 60%. Posaconazole has therapeutic advantages distinguishing it from other agents in terms of the prevention of invasive fungal infections. Posaconazole has broad-spectrum antifungal activity. In vitro and in vivo experiments have proven that posaconazole is useful in preventing or treating various fungal infections, such as infections caused by the following fungi: *Candida, dermatophytes, Dimorphics, Dematiaceous* (such as *Alternaria* and *Bipolaris), Aspergillus, Acremonium, Basidiomycetes, Bjerkandera, Coprinus, Paecilomyces, Microsporum, Trichophyton, Pseudallescheria, Schizophyllum, Crytococcus, Histoplasma, Blastomyces, Coccidioides, Fusarium, Exophiala, Mucor, Rhizopus* and *Rhizomucor, Kluyveromyces, Saccharomyces, Yarrowia, Pichia, Epidermophyton, Paracoccidioides, Scedosporium, Apophysomyces, Curvularia, Penicillium, Fonsecaea, Wangiella, Sporothrix, Pneumocystis, Trichosporon, Absidia, Cladophialophora, Ramichloridium, Syncephalastrum, Madurella, Scytalidium, Leshmania.* Moreover, posaconazole may also be used for infections caused by protists or bacteria.

The main mechanism of action of posaconazole is to act by reducing the synthesis of ergosterol. Ergosterol is a key substance involved in the synthesis process of fungal cells, participates in the synthesis of some important proteins in the cells, and is necessary for fungal cells. Posaconazole inhibits lanosterol 14α-demethylase (P45014DM) in fungi, reduces its activity, and causes lanosterol accumulation and ergosterol deficiency in the cells, resulting in the inability of cell membrane synthesis, thereby exerting its antifungal effects.

Posaconazole was first approved by the FDA in September 2006, and launched by Merck Sharp & Dohme under the trade name Noxafil^{®}. The currently approved dosage forms include injections, suspensions and enteric-coated tablets. Posaconazole is indicated for: (1) prophylaxis of invasive *Aspergillus* and *Candida* infections in patients 13 years of age and older who are at high risk of developing these infections due to being severely immunocompromised, such as hematopoietic stem cell transplantation (HSCT) recipients with graft-versus-host disease (GVHD) or those with hematologic malignancies with prolonged neutropenia from chemotherapy; (2) treatment of oropharyngeal candidiasis, including oropharyngeal candidiasis refractory to itraconazole and/or fluconazole.

Posaconazole has poor water solubility and is soluble at low pHs. It has a solubility of about 0.8 mg/mL in the gastric environment with about pH 1, and has a solubility of less than about 1 µg/mL at about pH 6.4 or higher. Therefore, when posaconazole dissolved in gastric fluid enters the intestines, a large amount of precipitation occurs, leading to a lower bioavailability of posaconazole formulations and a significant inter-individual variability. This poses a challenge in the development of posaconazole formulations, especially those for oral administration.

Taking Noxafil^{®} posaconazole oral suspension, which was approved by the FDA in 2006, as an example, uncontrollable and unpredictable precipitation occurs when posaconazole enters the small intestine in a fasting state, resulting in low absorption of posaconazole and high inter-individual variability when posaconazole is administered orally. To ensure an effective blood concentration, the oral suspension needs to be administered several times a day together with food (preferably high-fat food) or nutritional supplements to produce a sufficient plasma concentration of posaconazole. For example, the plasma concentration is 4 times higher when posaconazole is administered with a high fat food to a patient, and 3 times higher when posaconazole is administered with nutritional supplements to a patient, than administration to a fasted patient. The requirements of multiple administrations per day and administration with food result in poor patient compliance and limit the use of posaconazole oral suspension.

To overcome the above limitations of posaconazole oral suspension, Merck Sharp & Dohme developed Noxafil^{®} posaconazole enteric-coated tablets prepared by hot melt extrusion process, which was approved by the FDA in 2013. The bioavailability of this enteric-coated tablet is three times higher than that of Noxafil^{®} oral suspension. However, the hot melt extrusion process for producing the Noxafil^{®} posaconazole enteric-coated tablets is very complex. There are very few suppliers of the equipment used, and the equipment is expensive, has low production efficiency and low output. In addition, the instructions for the tablets also clearly state that posaconazole enteric-coated tablets should be swallowed whole and should not be divided, crushed or chewed, and the tablets should still be administered with food.

Noxafil^{®} posaconazole injection was approved by FDA in 2014, which enabled the injection of posaconazole for the first time and the use for patients who cannot take oral medications or are in critical condition. However, this injection also has significant drawbacks that limit its clinical application: (1) the formulation contains a large amount of sulfobutyl-β-cyclodextrin, which has toxic side effects; (2) the route of intravenous administration is central vein administration, resulting in poor safety and cumbersome operation; (3) large particles may occur in the formulation during administration, which requires an online filter and the administration must be carried out through a 0.22-micron polyethersulfone (PES) or polyvinylidene fluoride (PVDF) filter.

Researchers have been trying to solve the oral administration problem of posaconazole in order to find a better posaconazole formulation. For example, the Chinese Patent Application No. 200980122487.8 (corresponding to WO2009/129300) discloses an oral pharmaceutical composition containing posaconazole and HPMCAS in the form of a solid dispersion, which is intended to reduce the inter-individual variability of posaconazole in pharmacokinetic parameters. The solid dispersion is also prepared by hot melt extrusion. The required process is very complex and difficult to convert into industrial production. The Chinese Patent Application No. 201610839735.2 discloses a solid dispersion containing posaconazole and HPMCAS, which exhibited improved bioavailability and extended half-life of posaconazole than Noxafil^{®} suspension. However, no attention was paid to the impact of food. Therefore, there is still a need to develop posaconazole formulations with improved pharmacokinetic properties, simple preparation method, and convenient administration.

The posaconazole solid dispersion of the present disclosure is used for oral administration, has good dissolution and high bioavailability, and can be formulated into dispersible tablets, which is suitable for patients of all ages in different conditions, such as the elderly and frail, those with swallowing dysfunction, as well as infants and young children.

### Summary of Invention

In one aspect, the present disclosure provides a posaconazole solid dispersion comprising posaconazole, sodium dodecyl sulfate (SDS), silicon dioxide and hydroxypropyl methylcellulose acetate succinate (HPMCAS).

The inventors surprisingly found that the use of a specific ratio of SDS, silicon dioxide, and HPMCA can effectively improve the solubility and bioavailability of the poorly soluble drug posaconazole. Based on this finding, the present disclosure provides a posaconazole solid dispersion, which has significant advantages in terms of in vitro dissolution and high bioavailability. The significantly improved bioavailability than the commercially available formulations allows posaconazole to be used at a lower dose and for longer administration duration.

In another aspect, the present disclosure provides a method for preparing the posaconazole solid dispersion, which is simple and convenient, and can be used in large-scale production.

In another aspect, the present disclosure provides a posaconazole solid dispersion as described herein for use in the treatment or prevention of infections, particularly fungal infections.

In another aspect, the present disclosure provides a method of treating or preventing infections, particularly fungal infections, comprising administering to a patient an effective amount of a posaconazole solid dispersion as described herein.

Preferably, the fungal infection is invasive *Aspergillus* and *Candida* infection, such as oropharyngeal candidiasis, including oropharyngeal candidiasis refractory to itraconazole and/or fluconazole.

### Detailed description of Invention

In the first aspect, the present disclosure provides a posaconazole solid dispersion comprising posaconazole, silicon dioxide, sodium dodecyl sulfate (SDS) and hydroxypropyl methylcellulose acetate succinate (HPMCAS), wherein the weight ratio of SDS to posaconazole is ≥1:10, the weight ratio of posaconazole to silicon dioxide is 1:0.8 to 1:1, and the weight ratio of posaconazole to HPMCAS is 1:2 to 1:5.

The term "silicon dioxide" used herein is an inorganic porous material, also known as silicon dioxide nanoparticles, which is a silicon dioxide particle with a pore size of 2-50 nm. Silicon dioxide has a large specific surface area, high porosity, easy modification, adjustable pore size, narrow particle size distribution and good biocompatibility, and is an excellent drug carrier. Silicon dioxide can be prepared by a method known in the art, for example, by the method described in the Chinese patent ZL201710379143.1. Silicon dioxide is also commercially available, for example, from Fuji Silysia Chemical Ltd, Japan. In a preferred embodiment, silicon dioxide is selected from SYSLSIA350FCP, SYLORD244FP, SYLORD3050 and a mixture thereof in any ratio.

The term "hydroxypropyl methylcellulose acetate succinate" as used herein is a cellulose polymer. It is a white or off-white substance with a slightly acetic smell or no smell. In a preferred embodiment, hydroxypropyl methylcellulose acetate succinate is selected from HPMCAS-MG, HPMCAS-LF, HPMCAS-MF, HPMCAS-LG and a mixture thereof in any ratio.

In one embodiment, the weight ratio of SDS to posaconazole is 1:10 to 5:10, preferably 1:10 to 3:10, more preferably 1:10 to 2:10.

In one embodiment, the weight ratio of posaconazole to HPMCAS is 1:3 to 1:4.

In one embodiment, the weight ratio of posaconazole to silicon dioxide to HPMCAS is 1:1:1, 1:1:2, 1:1:3, 1:1:4, or 1:1:5.

In one embodiment, the posaconazole solid dispersion in accordance with the invention is prepared into tablets, which optionally further comprise filler, disintegrant, binder and/or lubricant. In a preferred embodiment, the posaconazole tablets in accordance with the invention further comprise microcrystalline cellulose, croscarmellose sodium and sodium stearyl fumarate in a weight ratio of 15:10:0.5. In a more preferred embodiment, the weight ratio of (posaconazole + SDS + silicon dioxide + HPMCAS) : microcrystalline cellulose : croscarmellose sodium : sodium stearyl fumarate is 100:15:10:0.5.

In one embodiment, the posaconazole solid dispersion in accordance with the invention is formulated into tablets, preferably dispersible tablets, which can be administered directly to a patient or can be administered to a patient after disintegrating in water. Preferably, the tablets are dispersible tablets, which are administered to a patient after disintegrating in water. The tablets prepared with the posaconazole solid dispersion in accordance with the invention can quickly disintegrate in water (≤3 minutes) to form a solution, and thus are applicable to patients of all ages and in different states, such as the elderly and frail, those with swallowing dysfunction, infants and young children or the like.

In one embodiment, the posaconazole dispersible tablets in accordance with the invention disintegrate in water within 20 seconds.

In the second aspect, the present disclosure provides a method for preparing the posaconazole solid dispersion in accordance with the invention, including the steps of:
(1) dissolving SDS in a solvent, then adding posaconazole to dissolve completely, and finally adding silicon dioxide and hydroxypropyl methylcellulose acetate succinate dissolved in a solvent, stirring evenly, to give a suspension, wherein the solvent is selected from 80.0%-92.5% aqueous ethanol solutions;
(2) drying the suspension obtained in step (1) to remove the solvent;
(3) collecting the solid obtained in step (2), crushing, and sieving, to give a solid dispersion in the form of granules.

In the third aspect, the present disclosure provides a method for preparing posaconazole tablets, including the steps of:
(1) dissolving SDS in a solvent, then adding posaconazole to dissolve completely, and finally adding silicon dioxide and hydroxypropyl methylcellulose acetate succinate dissolved in a solvent, stirring evenly, to give a suspension, wherein the solvent is selected from 80.0%-92.5% aqueous ethanol solutions;
(2) drying the suspension obtained in step (1) to remove the solvent;
(3) collecting the solid obtained in step (2), crushing, and sieving, to give a solid dispersion in the form of granules;
(4) compressing the granules obtained in step (3) into tablets, optionally adding filler, disintegrant and/or binder and optionally adding lubricant to the granules obtained in step (3) before compressing.

In the above step (1), there is no strict limitation on the amount of solvent used, provided that it is an amount in which posaconazole is fully dissolved.

In step (2), there is no strict limitation on the means and conditions of drying, provided that the solvent can be removed. The inventors have confirmed through experiments that the conventional drying method under reduced pressure can effectively remove the solvent used in the present invention without affecting the dissolution profile of posaconazole from the dispersion in accordance with the invention. In one embodiment, the drying in step (2) is carried out under a reduced pressure (-0.09 mPa or lower, for example -0.1 mPa to -0.09 mPa) at a temperature of 55-120°C, for example 70°C-110°C. In one embodiment, the drying in step (2) is carried out using a rotary evaporator. In another embodiment, the drying in step (2) is carried out using a belt dryer or a double cone tumble dryer. In industrial production, the belt dryer which has a high ethanol recovery rate, the highest drying efficiency and the highest production efficiency is most preferred. For example, when a belt dryer is used for drying, the temperature for the first stage can be set to 110°C, the temperature for the second stage can be set to 90°C, and naturally cooling is used for the third and fourth stages.

In step (3), there is no strict limitation on the conditions for crushing and sieving, provided that the particle size of the solid is appropriately reduced. In one embodiment, in step (3), the solid is passed through a 60-mesh sieve after crushing.

The preparation method of posaconazole in accordance with the invention is simple, has parameters easy to control, high yield and good reproducibility, and is easy to industrialize.

In the fourth aspect, the present disclosure provides a posaconazole solid dispersion as described herein for use in the treatment or prevention of fungal, bacterial and protistan infections, particularly fungal infections. Preferably, the fungal infection is invasive *Aspergillus* and *Candida* infection, such as oropharyngeal candidiasis, including oropharyngeal candidiasis refractory to itraconazole and/or fluconazole. For example, the posaconazole solid dispersion in accordance with the invention is used to prevent invasive *Aspergillus* and *Candida* infection and to treat oropharyngeal candidiasis, including oropharyngeal candidiasis refractory to itraconazole and/or fluconazole. More preferably, the posaconazole solid dispersion in accordance with the invention is used to prevent invasive *Aspergillus* and *Candida* infection.

In the fifth aspect, the present disclosure provides a method of treating or preventing fungal, bacterial and protistan infection, particularly fungal infection, comprising administering to a patient a therapeutically effective amount of a posaconazole solid dispersion in accordance with the invention. Preferably, the fungal infection is invasive *Aspergillus* and *Candida* infection, such as oropharyngeal candidiasis, including oropharyngeal candidiasis refractory to itraconazole and/or fluconazole.

In the sixth aspect, the present disclosure provides use of the posaconazole solid dispersion in accordance with the invention in the manufacture of a medicament for treating or preventing fungal, bacterial and protistan infection, particularly fungal infection; preferably, the fungal infection is invasive *Aspergillus* and *Candida* infection, such as oropharyngeal candidiasis, including oropharyngeal candidiasis refractory to itraconazole and/or fluconazole.

The posaconazole solid dispersion in accordance with the invention has significantly improved dissolution in various dissolution media, and exhibits a higher *in vivo* exposure, improved bioavailability and extended duration of action of posaconazole in Beagle dogs than the commercially available formulation Noxafil^{®} Posaconazole enteric-coated tablets (100 mg strength, Merck Sharp & Dohme Ltd (United Kingdom)).

The filler as described herein may be selected from microcrystalline cellulose, lactose, pregelatinized starches, starches, powdered sugar, and the like.

The disintegrant as described herein may be selected from sodium carboxymethyl starch, croscarmellose sodium, crospovidone, low-substituted hydroxypropyl cellulose, dry starches, and the like.

The binder as described herein may be selected from povidones such as povidone K30, dextrin, and the like.

The lubricant as described herein may be selected from magnesium stearate, sodium stearyl fumarate, and the like.

The term "AUCₗₐₛₜ" as used herein refers to the area under the plasma concentration-time curve from post-dose to the last blood collection point measured by the trapezoidal method, ng·h·ml⁻¹.

The term "AUC_{Inf}" as used herein refers to the measured area under the plasma concentration-time curve from 0 h extrapolated to infinity, ng·h·ml⁻¹.

The term "Cₘₐₓ" as used herein refers to the maximum plasma concentration of a drug after administration, ng/ml.

The term "Tₘₐₓ" as used herein refers to the time to the occurrence of Cₘₐₓ after administration, hour (h).

The terms "patient" as used herein refer to mammals and non-mammals in need of the posaconazole solid dispersion in accordance with the invention. The mammal means any member of the Mammalia class, which includes, but is not limited to: humans, non-human primates such as chimpanzees, and other apes and monkey species; farm animals such as cattle, horses, sheep, goats, swine; domestic animals such as rabbits, dogs, and cats; laboratory animals including rodents such as rats, mice and guinea pigs, and the like. Examples of non-mammal include, but are not limited to, birds. The term "patient" does not limit a specific age or gender. In some embodiments, the "patient" is a human.

The term "effective amount" as used herein refers to an amount of posaconazole effective in treating or preventing infection. For the posaconazole solid dispersion in accordance with the invention, the effective amount of posaconazole may be, for example, 100 mg to 400 mg, and may be administered once or several times per day. An amount outside the range may be used, if desired. The effective amount of posaconazole depends on factors such as the disease to be treated or prevented, the severity of the disease, age, weight and general health of the patient, as well as concomitant medications. The effective amount for a specific patient can be readily determined by the attending physician or veterinarian.

The term "absolute ethanol" as used herein refers to ethanol with a purity of not less than 99.5%.

The terms "comprise", "contain", "include" and their variants as used herein mean that in addition to the specified elements, other elements may be included, and also cover "consist of" the specified elements.

The technical and scientific terms as used herein which have not been specifically defined have the meaning commonly understood by a skilled person in the field to which the present invention belongs.

### Brief Description of the Drawings

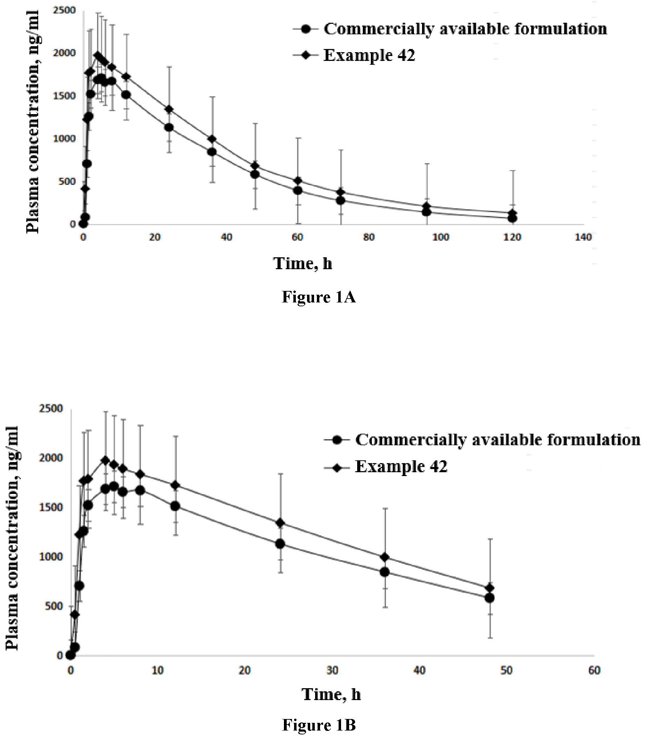

Figure 1A shows the pharmacokinetic curve of single oral administration of the tablets of Example 42 of the present application and the commercially available formulation Noxafil^{®} posaconazole enteric-coated tablets to Beagle dogs in fasted state at a dose of 100 mg per dog. Figure 1B is an enlarged partial view of Figure 1A.

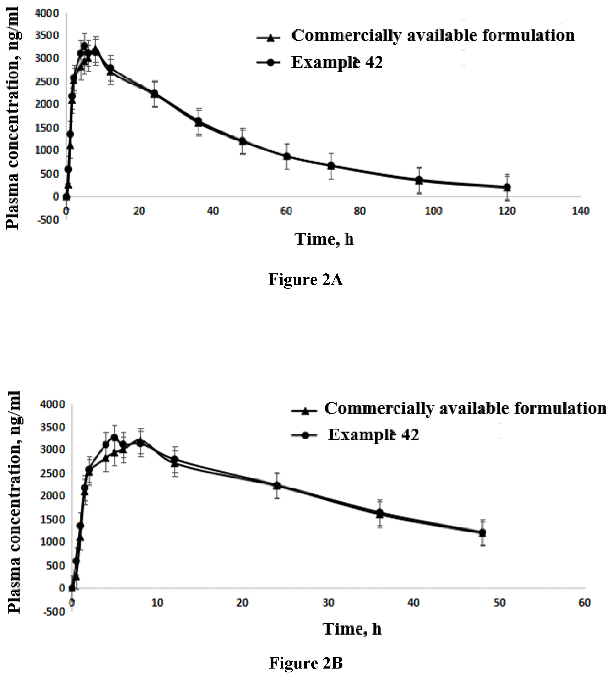

Figure 2A shows the pharmacokinetic curve of single oral administration of the tablets of Example 42 of the present application and the commercially available formulation Noxafil^{®} posaconazole enteric-coated tablets to Beagle dogs in fed state at a dose of 100 mg per dog. Figure 2B is an enlarged partial view of Figure 2A.

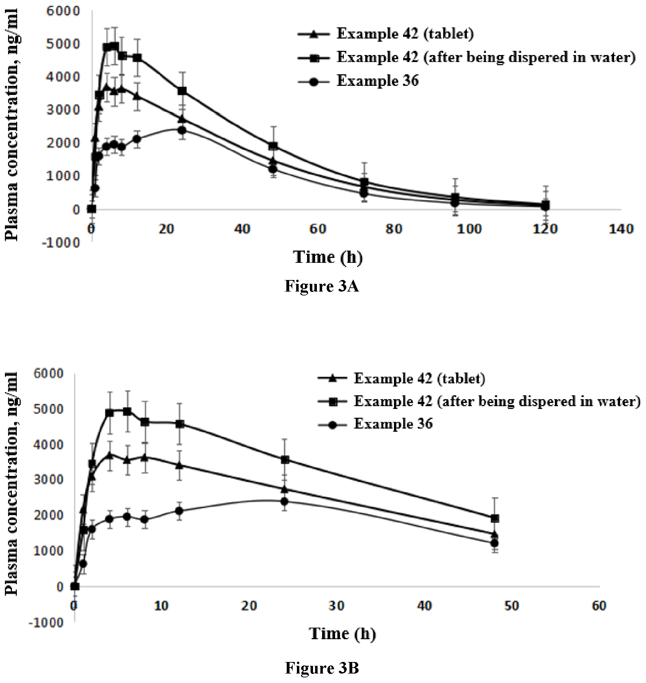

Figure 3A shows the pharmacokinetic curve of single oral administration of the tablets of Example 42 of the present application, the suspension obtained by dispersing the tablets of Example 42 of the present application in water, and the tablets of Example 36 of the present application to Beagle dogs in a fasted state at a dose of 200 mg per dog. Figure 3B is an enlarged partial view of Figure 3A.

### Examples

The following examples further illustrate the present invention, but not limit the scope of the appended claims.

Unless specified otherwise, the silicon dioxide SYSLSIA350FCP, SYSLSIA770FCP, SYLORD244FP and SYLORD 3050 used in the Examples were purchased from Fuji Silysia Chemical Ltd, Japan and Grace GmbH & Co. KG; the hydroxypropyl methylcellulose acetate succinate HPMCAS-LF, HPMCAS-MF, HPMCAS-HF, HPMCAS-LG, HPMCAS-MG, HPMCAS-HG used in the Examples were purchased from Shin-Etsu Chemical Co., Ltd., Japan. Example 1: Using 95% aqueous ethanol as solvent

1 g of posaconazole was weighed and taken up in 200ml of absolute ethanol under stirring. It was found that Posaconazole could not be completely dissolved after 3 hours.

1 g of posaconazole was weighed and taken up in 250ml of absolute ethanol under stirring. Posaconazole was completely dissolved after 1 hour. 3 g SYSLSIA350FCP was added and mixed well under stirring. 2 g HPMCAS-MG was added, and could not be completely dissolved after stirring for 2 hours.

The experiment was carried out by replacing the solvent with 95% aqueous ethanol. 1 g of posaconazole was weighed and taken up in 200ml of 95% aqueous ethanol under stirring. Posaconazole was completely dissolved after 45 minutes.

2 g of posaconazole was weighed and taken up in 200ml of 95% aqueous ethanol under stirring. Posaconazole cannot be completely dissolved after 3 hours.

In order to improve the solubility of the API, the surfactant SDS was added to the formulation. 0.2 g SDS was weighed and dissolved in 100 ml of 95% aqueous ethanol under stirring. 2 g of posaconazole was added, stirred, and completely dissolved after 30 minutes. 6 g SYSLSIA350FCP was added under stirring. Then, 4 g HPMCAS-MG dissolved in 100 ml of 95% aqueous ethanol was added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 2: Using 92.5% aqueous ethanol as solvent

0.2 g SDS was weighed and dissolved in 100 ml of 92.5% aqueous ethanol under stirring. 2 g of posaconazole was added, stirred, and completely dissolved after 15 minutes. 6 g SYSLSIA350FCP and then 4 g HPMCAS-MG dissolved in 100 ml of 92.5% aqueous ethanol were added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 3: Using 90% aqueous ethanol as solvent

0.2 g SDS was weighed and dissolved in 100 ml of 90% aqueous ethanol under stirring. 2 g of posaconazole was added, stirred, and completely dissolved after 10 minutes. 6 g SYSLSIA350FCP and then 4 g HPMCAS-MG dissolved in 100 ml of 90% aqueous ethanol were added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 4: Using 85% aqueous ethanol as solvent

0.2 g SDS was weighed and dissolved in 100 ml of 85% aqueous ethanol under stirring. 2 g of posaconazole was added, stirred, and completely dissolved within 10 minutes. 6 g SYSLSIA350FCP and then 4 g HPMCAS-MG dissolved in 100 ml of 85% aqueous ethanol was added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 5: Using 80% aqueous ethanol as solvent

0.2 g SDS was weighed and dissolved in 100 ml of 80% aqueous ethanol. 2 g posaconazole was added, stirred, and completely dissolved within 10 minutes. 6 g SYSLSIA350FCP and then 4 g HPMCAS-MG dissolved in 100 ml of 80% aqueous ethanol was added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 6: Using 75% aqueous ethanol as solvent

0.2 g SDS was weighed and dissolved in 100 ml of 75% aqueous ethanol under stirring. 2 g of posaconazole was added, stirred, and completely dissolved within 10 minutes. 6 g SYSLSIA350FCP and then 4 g HPMCAS-MG dissolved in 100 ml of 75% aqueous ethanol were added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 7: Dissolution Measurement

Based on the solubility properties of posaconazole, the dissolutions of the granules of Examples 1-6 were measured in phosphate buffer, pH 6.8 as follows: according to the measurement method of dissolution and release rate (Method 2 (Paddle) under 0931, General Chapters, Pharmacopoeia of the People's Republic of China 2015), 250ml of phosphate buffer (9.0±0.1 g of sodium dihydrogen phosphate was taken, added with 1000ml of water to dissolve, added with 14.8 ml of Tween 80, and mixed evenly) was used as the dissolution medium, with a rotation speed of 75 rpm/min. 10 ml of the solution was taken at 5 min, 15 min, 30 min, 60 min, 120 min and filtered, and the filtrate was used as the test sample. Separately, 20 mg of reference standard posaconazole was taken, weighed accurately into a 200 ml volumetric flask. 10 ml of methanol was added and sonicated for 5 minutes to dissolve, diluted to the calibration mark with 0.01 mol/L hydrochloric acid solution, and shake evenly. The obtained solution was used as a reference solution. The measurement was carried out according to the High Performance Liquid Chromatography method (0512, General Chapters, Pharmacopoeia of the People's Republic of China 2015, Volume IV), using phenylsilane bonded silica gel as the filler (Agilent Zorbax SB-Phenyl, 150mm*4.6mm, 3.5 µm or a chromatographic column with equivalent performance) with water-acetonitrile-phosphoric acid (640:360:1.5) as the mobile phase, the column temperature of 40°C, the flow rate of 2.0ml/min, and the detection wavelength of 254 nm. 15 µl of the reference solution and the test solution were taken respectively and injected into the liquid chromatograph to record the chromatogram. The dissolutions of the solid dispersions of Examples 1-6 were calculated based on the peak area using the external standard method. The results are shown in Table 1.

**Table 1**

| Example No. | Dissolution (%) | | | | |
|---|---|---|---|---|---|
| | 5 min | 15 min | 30 min | 60 min | 120 min |
| 1 | 35 | 38 | 41 | 45 | 49 |
| 2 | 47 | 50 | 53 | 54 | 57 |
| 3 | 48 | 51 | 52 | 54 | 56 |
| 4 | 47 | 51 | 53 | 55 | 58 |
| 5 | 46 | 49 | 51 | 53 | 56 |
| 6 | 32 | 37 | 41 | 40 | 40 |

Examples 1-6 showed that the excipients could not be completely dissolved in absolute ethanol, and using ethanol at different concentrations could make the excipients dissolve and disperse better. The results in Table 1 showed that the dissolutions of Examples 2, 3, 4 and 5 were significantly better than those of Examples 1 and 6, suggesting that the solvent used in the preparation process affected the dispersion of the API and excipients to some extent under the circumstance that the components of the composition were substantively the same. The posaconazole solid dispersions prepared using 80.0% (v/v) to 92.5% (v/v) ethanol as a solvent had better dissolution.

### Example 8: The weight ratio of SDS to posaconazole is 0.5:10

0.1 g of SDS was weighed and dissolved in 100 ml of 92.5% aqueous ethanol under stirring. 2 g of posaconazole was added under stirring and completely dissolved in 30 minutes. 6 g of SYSLSIA350FCP and then 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 9: The weight ratio of SDS to posaconazole is 1.5:10

0.3 g of SDS was weighed and dissolved in 100 ml of 92.5% aqueous ethanol under stirring. 2 g of posaconazole was added under stirring and completely dissolved within 10 minutes. 6 g of SYSLSIA350FCP and then 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 10: The weight ratio of SDS to posaconazole is 2:10

0.4 g of SDS was weighed and dissolved in 100 ml of 92.5% aqueous ethanol under stirring. 2 g of posaconazole was added under stirring and completely dissolved within 10 minutes. 6 g of SYSLSIA350FCP and then 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 11: Dissolution Measurement

The dissolutions of the posaconazole solid compositions in the form of granules prepared in Examples 8-10 were measured according to the method described in Example 7, and the results are shown in Table 2.

**Table 2**

| Example No. | Dissolution (%) | | | | |
|---|---|---|---|---|---|
| | 5 min | 15 min | 30 min | 60 min | 120 min |
| 8 | 40 | 43 | 45 | 46 | 48 |
| 2 | 47 | 50 | 53 | 54 | 57 |
| 9 | 46 | 52 | 53 | 55 | 58 |
| 10 | 46 | 51 | 53 | 54 | 57 |

The above dissolution results of Examples 2 and 8-10 showed that the addition of SDS increased the solubility of posaconazole, shortened the dissolution time of posaconazole, and greatly reduced the solvent cost and production cost. When the ratio of SDS to posaconazole was 1:10 or higher, the dissolution was good and could satisfy the production needs. In order to reduce the material cost and maintain tablet weight, the ratio of SDS to posaconazole is preferably 1:10 w/w.

### Example 12. Using silicon dioxide SYSLSIA770FCP

0.2 g of SDS was weighed and dissolved in 100 ml of 92.5% aqueous ethanol under stirring. 2 g of posaconazole added and completely dissolved under stirring. Then, 6 g of SYSLSIA770FCP and finally 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 13. Using silicon dioxide SYLORD244FP

0.2 g of SDS was weighed and dissolved in 100 ml of 92.5% aqueous ethanol under stirring. 2 g of posaconazole added and completely dissolved under stirring. Then, 6 g of SYLORD244FP and finally 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 14. Using silicon dioxide SYLORD3050

0.2 g of SDS was weighed and dissolved in 100 ml of 92.5% aqueous ethanol under stirring. 2 g of posaconazole added and completely dissolved under stirring. Then, 6 g of SYLORD3050 was added and finally 4 g of HPMCAS-MG dissolved in 100 ml of 92.5% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 15. Using HPMCAS-LF

0.2 g of SDS was weighed and dissolved in 100 ml of 85% aqueous ethanol under stirring. 2 g of posaconazole added and completely dissolved under stirring. Then, 6 g of SYSLSIA350 FCP and finally 4 g of HPMCAS-LF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 16. Using HPMCAS-MF

0.2 g of SDS was weighed and dissolved in 100 ml of 85% aqueous ethanol under stirring. 2 g of posaconazole added and completely dissolved under stirring. Then, 6 g of SYSLSIA350FCP and finally 4 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 17. Using HPMCAS-HF

0.2 g of SDS was weighed and dissolved in 100 ml of 85% aqueous ethanol under stirring. 2 g of posaconazole added and completely dissolved under stirring. Then, 6 g of SYSLSIA350FCP and finally 4 g of HPMCAS-HF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 18. Using HPMCAS-HG

0.2 g of SDS was weighed and dissolved in 100 ml of 85% aqueous ethanol under stirring. 2 g of posaconazole was added and completely dissolved under stirring. 6 g of SYSLSIA350FCP and then 4 g of HPMCAS-HG dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 19. Using HPMCAS-LG

0.2 g of SDS was weighed and dissolved in 100 ml of 85% aqueous ethanol under stirring. 2 g of posaconazole was added and completely dissolved under stirring. Then, 6 g of SYSLSIA350FCP and finally 4 g of HPMCAS-LG dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 20. Using Eudragit^{®} L100-55

0.2 g of SDS was weighed and dissolved in 100 ml of 85% aqueous ethanol under stirring. 2 g of posaconazole was added and completely dissolve under stirring. Then, 6 g of SYSLSIA350FCP and finally 4 g of Eudragit^{®} L100-55 dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 21. Using Eudragit^{®} L100

0.2 g of SDS was weighed and dissolved in 100 ml of 85% aqueous ethanol under stirring. 2 g of posaconazole was added and completely dissolved under stirring. Then, 6 g of SYSLSIA350FCP and finally 4 g of Eudragit^{®}L100 dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 22. Use of HPMC HP-55

0.2 g of SDS was weighed and dissolved in 100 ml of 85% aqueous ethanol under stirring. 2 g of posaconazole was added and completely dissolved under stirring. Then, 6 g of SYSLSIA350FCP and finally 4 g of HPMC HP-55 dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 23. Dissolution Measurement

The dissolutions of the posaconazole solid compositions in the form of granules prepared in Examples 12-22 were measured according to the method described in Example 7, and the results are shown in Table 3.

**Table 3**

| Example No. | Dissolution (%) | | | | |
|---|---|---|---|---|---|
| | 5 min | 15 min | 30 min | 60 min | 120 min |
| 2 | 47 | 50 | 53 | 54 | 57 |
| 12 | 35 | 36 | 38 | 40 | 44 |
| 13 | 46 | 50 | 52 | 55 | 56 |
| 14 | 26 | 29 | 31 | 34 | 37 |
| 4 | 47 | 51 | 53 | 55 | 58 |
| 15 | 48 | 51 | 53 | 54 | 57 |
| 16 | 47 | 49 | 52 | 54 | 56 |
| 17 | 36 | 42 | 45 | 48 | 51 |
| 18 | 38 | 43 | 46 | 48 | 52 |
| 19 | 46 | 51 | 54 | 56 | 58 |
| 20 | 12 | 15 | 17 | 20 | 25 |
| 21 | 10 | 14 | 17 | 22 | 24 |
| 22 | 15 | 18 | 20 | 24 | 28 |

The results in Table 3 showed that the granules of Examples 2 and 13 had similar and good dissolutions, which were significantly better than the dissolutions of the solid dispersions of Examples 12 and 14, suggesting that different types of silicon dioxide affected the dissolution of posaconazole. The silicon dioxide SYSLSIA350FCP and SYLORD244FP used in Examples 2 and 13 are preferred. The dissolutions exhibited by using other types of silicon dioxide are not ideal.

In addition, the results in Table 3 also showed that the solid dispersions of Examples 4, 15, 16, and 19 had similar and good dissolutions, which were better than the dissolutions of the solid dispersions of Examples 17 and 18, and were significantly better than the dissolutions of the solid dispersions of Examples 20, 21 and 22, suggesting that these four specific types of hydroxypropyl methylcellulose acetate succinate, namely, HPMCAS-MG, HPMCAS-LF, HPMCAS-MF and HPMCAS-LG are more preferred for the dissolution of posaconazole. The dissolutions of granules prepared by using other types of HPMCAS such as HPMCAS-HF and HPMCAS-HG, the polyacrylate Eudragit^{®}, hydroxypropyl methylcellulose are not ideal.

### Example 24: The weight ratio of posaconazole: silicon dioxide: hydroxypropyl methylcellulose acetate succinate = 1:1:1

0.2 g of SDS was weighed and dissolved in 100 ml of 85% aqueous ethanol under stirring. 2 g of posaconazole was added and completely dissolved under stirring. Then, 2 g of SYSLSIA350FCP and finally 2 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 25: The weight ratio of posaconazole: silicon dioxide: hydroxypropyl methylcellulose acetate succinate = 1:1:2

0.2 g of SDS was weighed and dissolved in 100 ml of 85% aqueous ethanol under stirring. 2 g of posaconazole was added and completely dissolved under stirring. Then, 2 g of SYSLSIA350FCP and finally 4 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 26: The weight ratio of posaconazole: silicon dioxide: hydroxypropyl methylcellulose acetate succinate = 1:1:3

0.2 g of SDS was weighed and dissolved in 100 ml of 85% aqueous ethanol under stirring. 2 g of posaconazole was added and completely dissolved under stirring. Then, 2 g of SYSLSIA350FCP and finally 6 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 27: The weight ratio of posaconazole: silicon dioxide: hydroxypropyl methylcellulose acetate succinate = 1:1:4

0.2 g of SDS was weighed and dissolved in 100 ml of 85% aqueous ethanol under stirring. 2 g of posaconazole was added and completely dissolved under stirring. Then, 2 g of SYSLSIA350FCP and finally 8 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 28: The weight ratio of posaconazole: silicon dioxide: hydroxypropyl methylcellulose acetate succinate = 1:1:5

0.2 g of SDS was weighed and dissolved in 100 ml of 85% aqueous ethanol under stirring. 2 g of posaconazole was added and completely dissolved under stirring. Then, 2 g of SYSLSIA350FCP and finally 10 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 29: The weight ratio of posaconazole: silicon dioxide: hydroxypropyl methylcellulose acetate succinate = 1:2:2

0.2 g of SDS was weighed and dissolved in 100 ml of 85% aqueous ethanol under stirring. 2 g of posaconazole was added and completely dissolved under stirring. Then, 4 g of SYSLSIA350FCP and finally 4 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 30: The weight ratio of posaconazole: silicon dioxide: hydroxypropyl methylcellulose acetate succinate = 1:2:1

0.2 g of SDS was weighed and dissolved in 100 ml of 85% aqueous ethanol under stirring. 2 g of posaconazole was added and completely dissolved under stirring. Then, 4 g of SYSLSIA350FCP and finally 2 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 31: The weight ratio of posaconazole: silicon dioxide: hydroxypropyl methylcellulose acetate succinate = 1:2:3

0.2 g of SDS was weighed and dissolved in 100 ml of 85% aqueous ethanol under stirring. 2 g of posaconazole was added and completely dissolved under stirring. Then, 4 g of SYSLSIA350FCP and finally 6 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 32: The weight ratio of posaconazole: silicon dioxide: hydroxypropyl methylcellulose acetate succinate = 1:2:4

0.2 g of SDS was weighed and dissolved in 100 ml of 85% aqueous ethanol under stirring. 2 g of posaconazole was added and completely dissolved under stirring. Then, 4 g of SYSLSIA350FCP and finally 8 g of HPMCAS-MF dissolved in 100 ml of 85% aqueous ethanol were added into the original reaction vessel, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 33: The weight ratio of posaconazole: silicon dioxide: hydroxypropyl methylcellulose acetate succinate = 1:3:1

0.2 g of SDS was weighed and dissolved in 100 ml of 85% aqueous ethanol under stirring. 2 g of posaconazole was added and completely dissolved under stirring. Then, 6 g of SYSLSIA350FCP and finally 2 g of HPMCAS-MF dissolved in 100 ml of 85% aqueous ethanol were added, suspended for 15 min, and dried under reduced pressure at 70°C. The solvent was volatilized to give a white solid. The solid was crushed and passed through a 60-mesh sieve to give granules.

### Example 34: The weight ratio of posaconazole: silicon dioxide: hydroxypropyl methylcellulose acetate succinate = 1:3:3

0.2 g of SDS was weighed and dissolved in 100 ml of 85% aqueous ethanol under stirring. 2 g of posaconazole was added and completely dissolved under stirring. Then, 6 g of SYSLSIA350FCP and finally 6 g of HPMCAS-MF dissolved in 100 ml of 85% aqueous ethanol were added, suspended for 15 min, and dried using a rotary evaporator under the conditions of 70°C, -0.09 mPa and 100 rpm to volatilize the solvent to give a white solid. The solid was crushed and passed through a 60-mesh sieve to give granules.

### Example 35: Dissolution Measurement

Based on the solubility properties of posaconazole, the dissolutions of the granules of Examples 24-34 were measured in the media hydrochloric acid solution, pH 1.0 and phosphate buffer, pH 6.8 as follows: according to the measurement method of dissolution and release (Method 2 (Paddle) under 0931, General Chapters, Pharmacopoeia of the People's Republic of China 2020).

Hydrochloric acid solution, pH 1.0: 1000 ml of 0.01mol/L hydrochloric acid solution as the dissolution medium.

Preparation of phosphate buffer, pH 6.8: 250 ml of phosphate buffer (9.0±0.1 g of sodium dihydrogen phosphate was taken, added with 1000 ml of water to dissolve, added with 14.8 ml of Tween 80, and mixed evenly) was taken and mixed evenly.

The remaining procedures were the same as those described in Example 7. The results are shown in Table 4 and Table 5.

**Table 4**

| Example No. | Posaconazole : silicon dioxide : HPMCAS | Dissolution (%) in basic solution (phosphate buffer, pH 6.8) | | | | |
|---|---|---|---|---|---|---|
| | | 5 min | 15 min | 30 min | 60 min | 120 min |
| 4 | 1:3:2 | 47 | 51 | 53 | 55 | 58 |
| 24 | 1:1:1 | 14 | 20 | 25 | 31 | 36 |
| 25 | 1:1:2 | 61 | 67 | 67 | 68 | 67 |
| 26 | 1:1:3 | 85 | 93 | 93 | 93 | 92 |
| 27 | 1:1:4 | 93 | 98 | 97 | 97 | 97 |
| 28 | 1:1:5 | 56 | 85 | 94 | 96 | 96 |
| 29 | 1:2:2 | 67 | 62 | 62 | 66 | 71 |
| 30 | 1:2:1 | 12 | 17 | 19 | 22 | 28 |
| 31 | 1:2:3 | 77 | 88 | 89 | 87 | 88 |
| 32 | 1:2:4 | 73 | 77 | 78 | 80 | 79 |
| 33 | 1:3:1 | 8 | 12 | 15 | 29 | 25 |
| 34 | 1:3:3 | 72 | 82 | 81 | 83 | 81 |

**Table 5**

| Example No. | Posaconazole : silicon dioxide : HPMCAS | Dissolution (%) in acid solution (hydrochloric acid solution, pH 1.0) | | | | |
|---|---|---|---|---|---|---|
| | | 5 min | 15 min | 30 min | 60 min | 120 min |
| 4 | 1:3:2 | 43 | 44 | 45 | 46 | 45 |
| 24 | 1:1:1 | 27 | 28 | 27 | 28 | 26 |
| 25 | 1:1:2 | 24 | 25 | 23 | 24 | 24 |
| 26 | 1:1:3 | 12 | 12 | 13 | 13 | 15 |
| 27 | 1:1:4 | 8 | 8 | 9 | 10 | 12 |
| 28 | 1:1:5 | 7 | 8 | 8 | 7 | 7 |
| 29 | 1:2:2 | 33 | 34 | 33 | 35 | 34 |
| 30 | 1:2:1 | 40 | 42 | 41 | 40 | 43 |
| 31 | 1:2:3 | 21 | 22 | 24 | 23 | 23 |
| 32 | 1:2:4 | 28 | 27 | 27 | 28 | 29 |
| 33 | 1:3:1 | 52 | 53 | 54 | 53 | 54 |
| 34 | 1:3:3 | 35 | 36 | 34 | 35 | 35 |

The results in Table 4 showed that the solid dispersions of Example 24, Example 30 and Example 33, in which the weight ratio of posaconazole to hydroxypropyl methylcellulose acetate succinate is 1:1 exhibited very poor dissolutions. In contrast, the solid dispersions of Examples 4, 24-29, 31, 32, and 34, in which the weight ratio of posaconazole to hydroxypropyl methylcellulose acetate succinate is 1:2 to 1:5 all exhibited good dissolutions. The Examples in which the weight ratio of posaconazole to hydroxypropyl methylcellulose acetate succinate is 1:3 to 1:4 exhibited superior dissolutions.

As mentioned above, when posaconazole dissolved in the gastric fluid enters the intestines, a large amount of precipitant occurs, leading to lower bioavailability of posaconazole formulations and a significant difference between individuals. Therefore, high dissolution in acidic environment and low dissolution in basic environment are not conducive to improve the absorption and bioavailability of posaconazole. According to the results in Table 4 and Table 5, by comparing Examples 24, 30 and 33, Examples 4, 25 and 29, and Examples 26, 31 and 34, the inventors surprisingly found that the amount of silicon dioxide affected both the dissolution of posaconazole in the acidic environment of the stomach and the dissolution of posaconazole in the basic environment of the intestine. As the weight ratio of silicon dioxide increases, the dissolution of posaconazole in a basic solution tends to decrease, while the dissolution in an acidic solution tends to increase. This suggests that it is necessary to determine the appropriate ratio of posaconazole to silicon dioxide. If the proportion of silicon dioxide is too high, it will lead to low dissolution of posaconazole in basic environment and high dissolution in an acidic environment, which is deleterious to the purpose of improving the absorption and bioavailability of posaconazole in vivo. Moreover, more importantly, the inventors surprisingly found that the effects of the amount of silicon dioxide determined in the present application on the dissolution of posaconazole in the acidic environment of the stomach and on the dissolution of posaconazole in the basic environment of the intestine had a consistent beneficial impact on improving the bioavailability of posaconazole and reducing difference between individuals. Therefore, using the amount of silicon dioxide determined herein may result in a substantial increase in the bioavailability, reduction in difference between individuals and difference between fasting and fed states of the composition containing posaconazole.

### Example 36: The weight ratio of posaconazole: silicon dioxide: hydroxypropyl methylcellulose acetate succinate = 1:0:4

20 g of posaconazole and 2 g of SDS were weighed and dissolved in 1000 ml of 85% aqueous ethanol. 60 g of HPMCAS-MF dissolved in 1000 ml of 85% aqueous ethanol was added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules. 50 g of the prepared granules was taken, added with 7.5 g of microcrystalline cellulose, 5 g of croscarmellose sodium and 0.25 g of sodium stearyl fumarate, mixed evenly, and compressed into tablets. The hardness was 8-12 kg.

### Example 37: The weight ratio of posaconazole: silicon dioxide: hydroxypropyl methylcellulose acetate succinate = 1:0.5:4

20 g of posaconazole and 2 g of SDS were weighed and dissolved in 1000 ml of 85% aqueous ethanol. 10 g of SYSLSIA350FCP and then 60 g of HPMCAS-MF dissolved in 1000ml of 85% aqueous ethanol were added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules. 50 g of the prepared granules was taken, added with 7.5 g of microcrystalline cellulose, 5 g of croscarmellose sodium and 0.25 g of sodium stearyl fumarate, mixed evenly, and compressed into tablets. The hardness was 8-12 kg.

### Example 38: The weight ratio of posaconazole: silicon dioxide: hydroxypropyl methylcellulose acetate succinate = 1:0.8:4

20 g of posaconazole and 2 g of SDS were weighed and dissolved in 1000 ml of 85% aqueous ethanol. 16 g of SYSLSIA350FCP and then 60 g of HPMCAS-MF dissolved in 1000ml of 85% aqueous ethanol were added under stirring, suspended for 15 min, and dried using a rotary evaporator under the conditions of 70°C, -0.09 mPa and 100 rpm to volatilize the solvent to give a white solid. The solid was crushed and passed through a 60-mesh sieve to give granules. 50 g of the prepared granules was taken, added with 7.5 g of microcrystalline cellulose, 5 g of croscarmellose sodium and 0.25 g of sodium stearyl fumarate, mixed evenly, and compressed into tablets. The hardness was 8-12 kg.

### Example 39: The weight ratio of posaconazole: silicon dioxide: hydroxypropyl methylcellulose acetate succinate = 1:1:4

100 g of the granules prepared in Example 27 was taken, added with 15 g of microcrystalline cellulose, 10 g of croscarmellose sodium and 0.5 g of sodium stearyl fumarate, mixed evenly, and compressed into tablets. The hardness was 8-12 kg.

### Example 40: The weight ratio of posaconazole: silicon dioxide: hydroxypropyl methylcellulose acetate succinate = 1:2:4

100 g of the granules prepared in Example 32 was taken, added with 15 g of microcrystalline cellulose, 10 g of croscarmellose sodium and 0.5 g of sodium stearyl fumarate, mixed evenly, and compressed into tablets. The hardness was 8-12 kg.

### Example 41: Investigation of dissolution and disintegration time

The dissolutions of the posaconazole tablets prepared in Examples 36-40 were measured according to the method described in Example 7. The results are shown in Table 6.

**Table 6**

| Example No. | Dissolution (%) in the basic solution | | | | | Disintegration time |
|---|---|---|---|---|---|---|
| | 5 min | 15 min | 30 min | 60 min | 120 min | |
| 36 | 75 | 89 | 94 | 95 | 95 | 3.5 min |
| 37 | 78 | 88 | 93 | 94 | 95 | 1.5 min |
| 38 | 81 | 92 | 95 | 94 | 96 | 45S |
| 39 | 83 | 94 | 95 | 96 | 95 | 20S |
| 40 | 70 | 77 | 78 | 78 | 79 | 20S |

In Examples 36-40, the weight ratio of posaconazole to silicon dioxide is in the range of 1 : 0-2. The results in Table 6 showed that the addition of silicon dioxide was beneficial to the disintegration of tablets, and the addition of silicon dioxide shortened the disintegration time of tablets from a disintegration time satisfying the requirement for ordinary oral tablets (≤5 min) to a disintegration time satisfying the requirement for dispersible tablets (≤3min). Specifically, when the weight ratio of posaconazole to silicon dioxide is 1:0.8, the tablet disintegrates within 45 seconds; when the amount of silicon dioxide is further increased to the 1:1 weight ratio of posaconazole:silica, the tablet disintegrates within 20 seconds.

According to the above dissolution results of Examples 24-34, it can be seen that improper increase in the amount of silicon dioxide will reduce the dissolution of posaconazole in the basic environment of the intestine. Thus, based on the two properties of disintegration time and dissolution, the weight ratio of posaconazole to silicon dioxide is preferably 1:0.8 to 1:1.

### Example 42: Pilot scale-up

50 g of SDS was weighed and dissolved in 50 L of 92.5% aqueous ethanol under stirring. 500 g of posaconazole was added and dissolved under stirring. 2000 g of HPMCAS-MF was added and dissolved under stirring. Finally, 500 g of SYSLSIA350FCP was added, suspended for 30 min, and dried in a 6 m² belt dryer. The two layers of the belt dryer were fed together for drying. The temperature at the first stage was set to 110°C, the temperature at the second stage was set to 90°C, and natural cooling was used in the third and fourth stages by not setting a temperature. It took 1 hour of drying time to remove the solvent to give a white solid. The solid was crushed and passed through a 60-mesh sieve to give granules.

The obtained granules were evenly mixed with microcrystalline cellulose, croscarmellose sodium and sodium stearyl fumarate in a weight ratio of 100:15:10:0.5 for compression into tablets. The hardness was 8-12 kg.

### Example 43: Dissolution Measurement

The dissolution of the posaconazole tablets prepared in Example 42 was measured according to the method described in Example 7. The results are shown in Table 7.

**Table 7**

| Example No. | Dissolution (%) | | | | |
|---|---|---|---|---|---|
| | 5 min | 15 min | 30 min | 60 min | 120 min |
| Example 42 | 82 | 98 | 98 | 98 | 97 |

### Example 44: Pharmacokinetics study in Beagle dogs (part 1)

In this study, the sample of Example 42 was used for a four-cycle four-crossover test, with a washout period of 15 days. Twelve Beagle dogs (half male and half female, body weight of about 10 kg) were randomly divided into four groups, with 3 dogs in each group. The Beagle dogs were orally administered once with the posaconazole formulation of Example 42 at a dose of 100 mg per dog or the commercially available enteric-coated tablet Noxafil^{®} at a dose of 100 mg per dog.

**Table 8. Dosing regimen for pharmacokinetics study in Beagle dogs**

| Cycles | Fasting | Group (n=3) |
|---|---|---|
| Cycle 1 | No | I test formulation |
| | No | II innovator drug as control |
| | Yes | III test formulation |
| | Yes | IV innovator drug as control |
| Cycle 2 | No | I innovator drug as control |
| | No | II test formulation |
| | Yes | III innovator drug as control |
| | Yes | IV test formulation |
| Cycle 3 | Yes | I test formulation |
| | Yes | II innovator drug as control |
| | No | III test formulation |
| | No | IV innovator drug as control |
| Cycle 4 | Yes | I innovator drug as control |
| | Yes | II test formulation |
| | No | III innovator drug as control |
| | No | IV test formulation |

1.5-2.0 ml of blood was collected from the small saphenous vein of the foreleg before administration (0h) and 0 min, 30 min, 1 h, 1.5 h, 2 h, 4 h, 6 h, 8 h, 12 h, 24 h, 36 h, 48 h, 60 h, 72 h, 96 h, 120 h after administration, respectively. The blood sample was placed in a heparinized EP tube immediately after being taken, centrifuged at 4000 rpm for 10 min to separate the plasma, which was frozen at -80°C for testing. After thawing, 50 µL of plasma was accurately aspirated into a 2 mL centrifuge tube with a stopper. 50 µL of internal standard solution (5 ng/mL dexamethasone in acetonitrile) and 200 µL of acetonitrile were precisely added, vortexed for 2 min to mix evenly, and centrifuged at 12,000 rpm for 10 minutes. The supernatant was analyzed by mass spectrometry-high performance liquid chromatography.

Mass spectrometry conditions: mass spectrometer model: AB LCMS-4000 tandem mass spectrometer (Serial No. L20584900322SS); polarity: positive ion mode
Chromatographic conditions:
Column: Waters XSELECT HSS T3 2.5µm 2.1×50mm;
Injection volume: 15 µL; flow rate: 0.6 mL/min; the gradient elution is performed as shown in Table 9;
Mobile phase: Solution A: 95% water (0.1% formic acid, 5% water)
Solution B: 95% acetonitrile (0.1% formic acid, 5% acetonitrile)

**Table 9 Gradient elution**

| Time (min) | Module | Events | Parameter |
|---|---|---|---|
| 0.20 | Pumps | PumpBConc. | 5.00 |
| 1.50 | Pumps | PumpBConc. | 95.0 |
| 1.80 | Pumps | PumpBConc. | 95.0 |
| 1.81 | Pumps | PumpBConc. | 5.00 |
| 2.30 | Pumps | PumpBConc. | 5.00 |

The blood concentration-time curves are shown in Figures 1 and 2, and the main pharmacokinetic parameters are shown in Table 10. WinNonlin software was used to calculate the pharmacokinetic parameters.

**Table 10. Comparison of pharmacokinetic parameters for Beagle dogs in fasting state**

| Pharmacokinetic parameters | Example 42 | Noxafil^{®} | Ratio (%) |
|---|---|---|---|
| Number of animals | 12 | 12 | - |
| Tₘₐₓ (h) | 3 | 2 | - |
| Cₘₐₓ (ng/mL) | 1962±865 | 1589±986 | 123.5 |
| AUCₗₐₛₜ (ng·h·ml⁻¹) | 75501±36598 | 58979±38976 | 128.0 |
| AUC_{Inf} (ng·h·ml⁻¹) | 78879±37846 | 60729±39729 | 129.9 |

**Table 11. Comparison of pharmacokinetic parameters for Beagle dogs in fed state**

| Pharmacokinetic parameters | Example 42 | Noxafil^{®} | Ratio (%) |
|---|---|---|---|
| Number of animals | 12 | 12 | - |
| Tₘₐₓ (h) | 6 | 5 | - |
| Cₘₐₓ (ng/mL) | 3221±1215 | 3357±1309 | 96.0 |
| AUCₗₐₛₜ(ng·h·ml⁻¹) | 131456±46210 | 140487±44677 | 93.6 |
| AUC_{Inf}(ng·h·ml⁻¹) | 138296±67603 | 149586±67929 | 92.5 |

| Variation ratio compared to the fasting state | | | |
|---|---|---|---|
| Cₘₐₓ (ng/mL) | 64.2% | 111.3% | - |
| AUCₗₐₛₜ(ng·h·ml⁻¹) | 74.1% | 138.2% | - |
| AUC_{Inf}(ng·h·ml⁻¹) | 75.3% | 146.3% | - |

The results in Table 10 showed that in fasting state, the ratio of AUC ₗₐₛₜ of the posaconazole tablet of Example 42 of the present application (administered at a dose of 100 mg per dog) to that of commercially available enteric-coated tablet Noxafil^{®} (administered at a dose of 100 mg per dog) was 129.9%, and the ratio of Cₘₐₓ was 123.5%. It can be seen that the bioavailability of the posaconazole tablet of Example 42 of the present application is significantly improved compared to the enteric-coated tablet Noxafil^{®}.

The results in Table 11 showed that in fed state, the ratio of AUC ₗₐₛₜ of the posaconazole tablets of Example 42 of the present application (administered at a dose of 100 mg per dog) to that of the commercially available enteric-coated tablet Noxafil^{®} (administered at a dose of 100 mg per dog) was 93.6%, and the ratio of Cₘₐₓ was 96.0%. It can be seen that the posaconazole tablet of Example 42 of the present application is bioequivalent to the enteric-coated tablet Noxafil^{®}.

By comparing the results in Table 10 and Table 11, it can be seen that as compared with administration in fasting state, feeding resulted in a 64.2% variation ratio in Cₘₐₓ and a 74.5% variation ratio in AUCₗₐₛₜ for the tablet of Example 42 of the present application; a 111.3% variation ratio in Cₘₐₓ and a 138.2% variation ratio in AUCₗₐₛₜ for the commercially available formulation. It can be seen that the tablet of Example 42 of the present application is less affected by food.

### Example 45: Pharmacokinetics study in Beagle dogs (part 2)

In this study, the samples of Example 36 and Example 42 were used to carry out a three-cycle three-crossover test, with a washout period of 15 days. Twelve Beagle dogs (half male, half female, body weight of about 10 kg) were randomly divided into three groups, with 4 dogs in each group. The Beagle dogs were orally administered once with the posaconazole formulation of Example 36 at a dose of 200 mg per dog or the posaconazole formulation of Example 42 at a dose of 200 mg per dog.

**Table 12. Dosing regimen for pharmacokinetics study in Beagle dogs**

| Cycles | Fasting | Groups (n=3) |
|---|---|---|
| Cycle 1 | Yes | I Example 42 |
| | Yes | II Example 36 |
| | Yes | III Example 42 (administered after dispersing in water) |
| Cycle 2 | No | I Example 42 (administered after dispersing in water) |
| | No | II Example 42 |
| | Yes | III Example 36 |
| Cycle 3 | Yes | I Example 36 |
| | Yes | II Example 42 (administered after dispersing in water) |
| | No | III Example 42 |

Blood sample was collected before administration (0h) and 0 min, 1 h, 2 h, 4 h, 6 h, 8 h, 12 h, 24 h, 48 h, 72 h, 96 h, 120 h after administration, respectively. The treatment method for plasma was the same as described in Example 44.

Mass spectrometry conditions and chromatographic conditions: the same as described in Example 44.

The blood concentration-time curve is as shown in Figure 3, and the main pharmacokinetic parameters are as shown in Table 13. WinNonlin software was used to calculate the pharmacokinetic parameters.

**Table 13. Comparison of pharmacokinetic parameters for Beagle dogs in fasting state**

| Pharmacokinetic parameters | Example 36 | Example 42 | Example 42 (administered after dispersing in water) |
|---|---|---|---|
| Number of animals | 12 | 12 | 12 |
| Tₘₐₓ (h) | 24 | 6 | 6 |
| Cₘₐₓ (ng/mL) | 2782±1734 | 3742±1750 | 5092±978 |
| AUCₗₐₛₜ(ng·h·ml⁻¹) | 120240±45340 | 166704±52271 | 216456±35210 |
| AUC_{Inf}(ng·h·ml⁻¹) | 121576±45684 | 169114±52769 | 219721±36184 |
| Ratio of Cₘₐₓ | - | 135 | 183 |
| Ratio of AUCₗₐₛₜ | - | 139 | 180 |
| Ratio of AUC_{Inf} | - | 139 | 181 |

The results in Table 13 showed that in fasting state, the ratio of AUCₗₐₛₜ of the posaconazole tablet of Example 42 of the present application (administered at a dose of 200 mg per dog) to that of the posaconazole tablet of Example 36 of the present application (administered at a dose of 200 mg per dog) was 139%, and the ratio of Cₘₐₓ is 135%. It can be seen that the bioavailability of the posaconazole tablet of Example 42 of the present application is significantly improved compared to that of Example 36 of the present application.

In fed state, the ratio of AUCₗₐₛₜ of the posaconazole tablet of Example 42 of the present application (administered after dispersing in water) (administered at a dose of 200 mg per dog) to that of the posaconazole tablet of Example 42 of the present application (administered at a dose of 200 mg per dog) was 130%, and the ratio of Cₘₐₓ was 136%. It can be seen that administrating the tablet of Example 42 after being dispersed in water leads to a further significant increase in the bioavailability of posaconazole, compared with orally administering the tablet of Example 42.

The specific embodiments are provided herein only to illustrate the invention, and do not aim to limit the claimed scope of the invention. Based on the present disclosure, equivalent variants of the technical solutions of the present disclosure can be obviously obtained by those skilled in the art, and those equivalent variants are also within the scope of the present disclosure.

## Claims

1. A posaconazole solid dispersion comprising posaconazole, silicon dioxide, sodium dodecyl sulfate (SDS) and hydroxypropyl methylcellulose acetate succinate (HPMCAS), wherein,
the weight ratio of SDS to posaconazole is ≥ 1:10, the weight ratio of posaconazole to silicon dioxide is 1:0.8 to 1:1, and the weight ratio of posaconazole to HPMCAS is 1:2 to 1:5,
the silicon dioxide is selected from SYSLSIA350FCP, SYLORD244FP, SYLORD3050 and a mixture thereof in any ratio; the HPMCAS is selected from HPMCAS-MG, HPMCAS-LF, HPMCAS-MF, HPMCAS-LG and a mixture thereof in any ratio.

2. The posaconazole solid dispersion according to claim 1, wherein the weight ratio of SDS to posaconazole is 1:10 to 5:10, preferably 1:10 to 3:10, more preferably 1:10 to 2:10.

3. The posaconazole solid dispersion according to claim 1 or 2, wherein the weight ratio of posaconazole to HPMCAS is 1:3 to 1:4.

4. The posaconazole solid dispersion according to claim 1 or 2, wherein the weight ratio of posaconazole to silicon dioxide to HPMCAS is 1:1:1, 1:1:2, 1:1:3, 1:1:4 or 1:1:5.

5. The posaconazole solid dispersion according to any one of claims 1 to 4, wherein the dispersion is formulated in the form of tablet, which optionally further comprises microcrystalline cellulose, croscarmellose sodium and sodium stearyl fumarate in a weight ratio of 15:10:0.5.

6. The posaconazole solid dispersion according to claim 5, wherein the weight ratio of (posaconazole+SDS+silicon dioxide+HPMCAS): microcrystalline cellulose: croscarmellose sodium: sodium stearyl fumarate is 100:15:10:0.5.

7. The posaconazole solid dispersion according to claim 6, wherein the tablet is a dispersible tablet that disintegrates in water within 20 seconds to form a suspension.

8. The posaconazole solid dispersion according to any one of claims 5 to 7, wherein the posaconazole solid dispersion is administered to a patient in the form of the tablet or is administered to a patient after the table has disintegrated in water.

9. A method for preparing the posaconazole solid dispersion according to any one of claims 1 to 4, including the steps of:
(1) dissolving SDS in a solvent, then adding posaconazole to dissolve completely, and finally adding silicon dioxide and hydroxypropyl methylcellulose acetate succinate dissolved in a solvent, stirring evenly, to give a suspension, wherein the solvent is selected from 80.0%-92.5% aqueous ethanol solutions;
(2) drying the suspension obtained in step (1) to remove the solvent;
(3) collecting the solid obtained in step (2), crushing, and sieving, to give a solid dispersion in the form of granules.

10. A method for preparing the posaconazole solid dispersion according to any one of claims 5 to 8, including the steps of:
(1) dissolving SDS in a solvent, then adding posaconazole to dissolve completely, and finally adding silicon dioxide and hydroxypropyl methylcellulose acetate succinate dissolved in a solvent, stirring evenly, to give a suspension, wherein the solvent is selected from 80.0%-92.5% aqueous ethanol solutions;
(2) drying the suspension obtained in step (1) to remove the solvent;
(3) collecting the solid obtained in step (2), crushing, and sieving, to give a solid dispersion in the form of granules;
(4) compressing the granules obtained in step (3) into tablets, optionally adding the other components to the granules obtained in step (3) before compressing.

11. The method according to claim 9 or 10, wherein the drying in step (2) is carried out under reduced pressure at a temperature of 55-120°C, for example, 70-110°C.

12. The method according to claim 9 or 10, wherein the drying in step (2) is carried out using a belt dryer.

13. The posaconazole solid dispersion according to any one of claims 1 to 8, for use in the treatment or prevention of fungal, bacterial and protistan infection, particularly fungal infection; preferably, the fungal infection is invasive *Aspergillus* and *Candida* infection, such as oropharyngeal candidiasis, including oropharyngeal candidiasis refractory to itraconazole and/or fluconazole.
